Europäisches Patentamt

European Patent Office

Office européen des brevets

(11) Publication number: **0 320 267**
**A2**

## EUROPEAN PATENT APPLICATION

(21) Application number: 88311640.2

(22) Date of filing: 08.12.88

(51) Int. Cl.⁴: **C07K 3/28** , **C07K 15/14** , **C12N 5/00** , //A61K35/12

(30) Priority: 10.12.87 US 131188

(43) Date of publication of application:
**14.06.89 Bulletin 89/24**

(84) Designated Contracting States:
**AT BE CH DE ES FR GB IT LI LU NL SE**

(71) Applicant: **CELL-BIO GROUP, LTD**
**330 North Main Street P.O. Box 686**
**Doylestown Pennsylvania 18901(US)**

(72) Inventor: **Dinka, Steven K.**
**R.D. 1 P.O. Box 537 Sanatorium Ave.**
**Otisville New York 10963(US)**

(74) Representative: **West, Alan Harry et al**
**R.G.C. Jenkins & Co. 26 Caxton Street**
**London SW1H 0RJ(GB)**

(54) Glycoprotein growth modulation materials.

(57) The invention relates to the production of cell growth promoting and cell growth inhibiting materials comprising glycoproteins which can be removed from biological fluids with the use of lectins. The invention relates also to culture media including such growth modulating materials and cell growth enhancers produced by the lysis of red blood cells.

EP 0 320 267 A2

## GLYCOPROTEIN GROWTH MODULATION MATERIALS

This invention relates to cell growth modulating materials present in blood and other biological fluids. There are described herein compositions and methods for the preparation and the use of these materials for cell growth, cell proliferation enhancement of cell growth, cell growth control, diagnosis and clinical applications.

In order to study the physiology and growth of both normal and abnormal cells derived from tisse or blood of higher organisms, scientists have sought to grow these cells in vitro in chemically defined culture media. Nearly all cells grown in culture media, however, require "growth factors" in order to proliferate, i.e. grow and divide.

In general, serum is used to provide these "growth factors". Serum, however, is a very complex material containing many substances. Furthermore, the concentration of the "growth factors" in serum is very low, which makes their purification in viable form extremely difficult. See generally, S.K. Dinka; "Growth Regulation Factors in Serum Used For In Vitro Culture of Cells: Principles and Techniques; In:E. Kurstak; "Techniques in the Life Sciences, Cl. Setting Up and Maintenance of Tissue and Cell Cultures" 1-33, (1985).

Some growth factors affect specific cells while other growth factors cause growth of a broader spectrum of cell types. Numerous scientists have sought means to isolate and purify substances responsible for cell growth. Some substances have been chemically identified and are polypeptide or protein in nature. Purification of such growth factors, however, usually present in very low concentrations in blood, is extremely difficult. Techniques presently available for extraction of growth factors from these complex systems generally are not appropriate for processing large volumes of serum (D. Gospodarowicz and J. S. Moran, 45 Ann. Rev. Biochem. 531-588, 1976).

Methodologies available for the fractionation of proteinaceous growth factors from serum were reviewed in my earlier patents U.S. Pat. Nos. 4,189, 535 and 4,436, 816. The first patent teaches adsorption of serum proteins to anionic exchange resins followed by specific elution of growth promoting materials utilizing gradient salt or pH solutions. The second patent teaches the addition of perchloric acid to serum or plasma to obtain a precipitate, followed by extraction of the growth factors by increasing the pH of the solution.

One group of growth factors in blood are the somatomedins, a group of circulating peptides complexed with large protein(s) believed to be of liver origin that appear to be regulated by growth hormone. Somatomedins have been dissociated from the larger carrier proteins in acidic environments and it has been shown that active somatomedin molecules of less than 10,000 dalton molecular weight are present in soluble form in ultrafiltrates of human plasma after acidification (pH 2.3) with acetic acid (B. H. Ginsberg et al., 48(2) J. Clin. Endocrinology and Metabolism 43-49, 1979). Somatomedins are in plasma protein precipitates formed during Cohn fractionation and they are dissociated from the carrier proteins by further acidification. The acid dissociation of somatomedins has been utilized in several methodologies for the isolation of these growth promoters from Cohn-precipitated plasma protein. (H. Burgi et al, 121 Biochem, Biophys. Acta. 349-359, 1966; Knut Uthne, 175 Acta Endocrinology (Suppl.) 1-35, 1973; E. Rinderknecht and R. E. Humble, 73(7) Proc. Natl. Acad. Sci. U.S.A. 2365-2369, 1976).

During the blood coagulation process, growth factors are released from blood platelets, and some researchers have claimed that a major portion of serum growth promoters are of platelet origin (See, e.g., R. Ross and A. Vogel, 14 Cell 203-210, 1978). As a result of the fact that growth factors account for only a small percentage of all serum protein, investigators have isolated platelet growth factors from preparations of lysed washed platelets utilizing ion exchange chromatographic procedures. C. H. Heldin et al., in 109 Exp. Cell Res. 429-437, 1977, described two anionic fractions of 40,000 and less than 10,000 dalton molecular weight, respectively, and a heterogenous cationic fraction of from 25,000 to 35,000 dalton size possessing activity. Ross, et al (in Hormones and Cell Culture, Book A. Eds. G. H. Sato and R. Ross, pp. 3-16 (Cold Spring Harbor Laboratory, 1979)) isolated a cationic fraction of 10,000 to 30,000 molecular weight. Further processing resolved mitogenic activity to molecules of 16,000 to 18,000 daltons.

These platelet-derived growth factors have been used to promote cell growth. However, these growth factors must still be applied with platelet poor plasma in order to be effective.

Several groups of investigators agree blood contains more than a single growth factor. For this reason, it appears desirable to develop a practical method of separating the diverse group of molecules possessing mitogenic activity from other blood proteins. In addition, there are not only many areas of practical application in which there may be made beneficial substitution of purified growth materials for whole serum or plasma, but the availability of growth material would make new areas of application of cell growth regulation possible. Some of the areas in which the availability of purified growth materials would be highly advantageous include the study of neoplastic diseases, dwarfism, neurological development, cellular immunity, transplantation (tissue grafting), would healing, replacement of serum for virus vaccine production, production of specific biomolecules (e.g., urokinase, interferon, monoclonal antibodies, etc.), in animal husbandry for increasing and maintaining rapid growth and wherever serum or plasma is required for cell mitosis.

Increased knowledge about these "growth factors" would also advance our understanding of cell growth regulation. The availability of purified factors would further the study of neoplastic diseases, development and maturation, aging, wound healing and tissue regenerations as well as the whole field related to cell growth and growth control. These factors are essential in biotechnology using high eukaryotic cells, such as mammalian and chicken cells in the production of biomolecules, e.g. virus vaccines, tissue plasminogen activator (TPS), urokinase, monoclonal antibodies, etc. As recited above, they could also have an important role in animal husbandry for increasing and maintaining rapid growth.

Heretofore, researchers have concentrated on identifying growth factors which exist as smaller compounds in both animals and humans. The present invention results from the novel discovery that growth promoting and inhibitory substances exist in serum, plasma or other body fluids such as mother's milk, saliva and cerebrospinal fluid in the form of glycoproteins where the carbohydrate moiety and its configuration plays an essential role.

This invention relates to materials suitable for cell growth modulation (i.e. growth activation or inhibition) obtained from serum, plasma, or other body fluids, or a separated fraction extract thereof, preparation or product of these, as well as the processes for obtaining same.

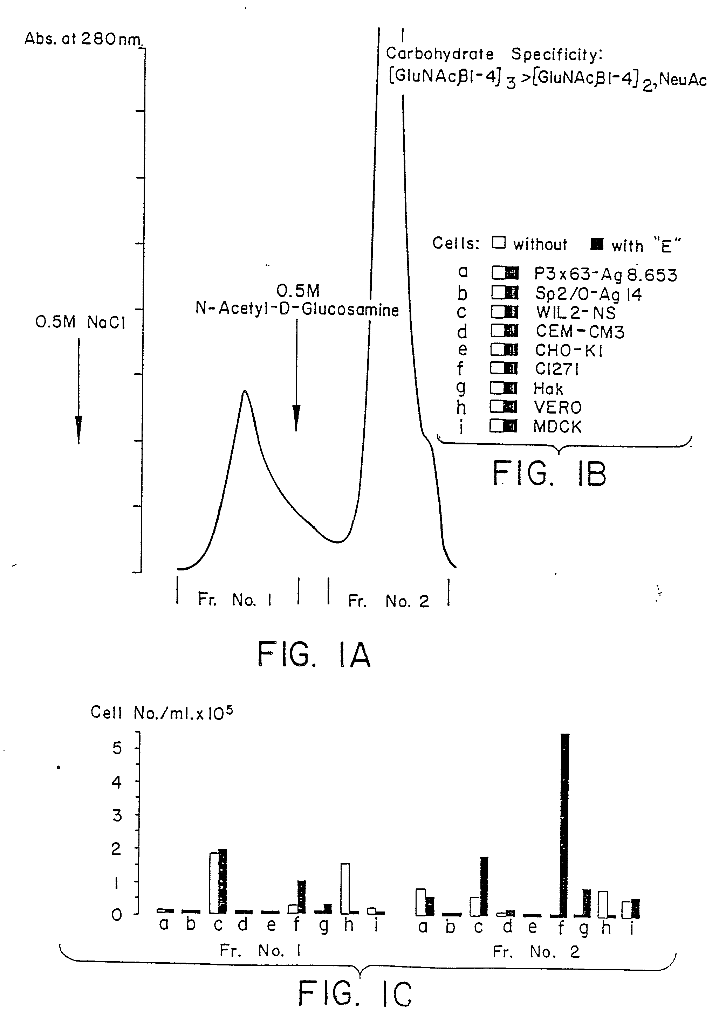

Fig. 1A through Fig. 1C represent the results obtained from the wheat germ lectin chromatography. Fig. 1A represents UV light absorption of the chromatogram for a test sample at 280 nm. The graph shows two fractions labelled as Fr. No. 1 and Fr. No. 2. These fractions were isolated and collected, dialyzed against physiological saline and filtered through a 0.2 micron sterile filter.

Fig. 1B shows nine different cell lines which were used in the test for growth promotion. The clear portion represents cell growth in media without cell growth enhancer "E" and the darkened field represents cell growth in media with cell growth enhancer "E".

Fig. 1C is a bar graph representing the cell growth of the nine cell lines of Fig. 1B grown with Fr. No. 1 and Fr. No. 2 (see Fig. 1A). The cell lines were incubated at 37° C for an appropriate period of time and then the number of cells x$10^5$ per ml were counted. The cell lines (a through i) in each of Fr. No. 1 and Fr. No. 2 in Fig. 1C from left to right correspond to the cell lines (a through i) in Fig. 1B from top to bottom, respectively. As in Fig. 1B, the light areas of the bar graph in Fig. 1C correspond to cell growth in media without cell growth enhancer "E" and the dark areas correspond to cell growth in media with cell growth enhancer "E".

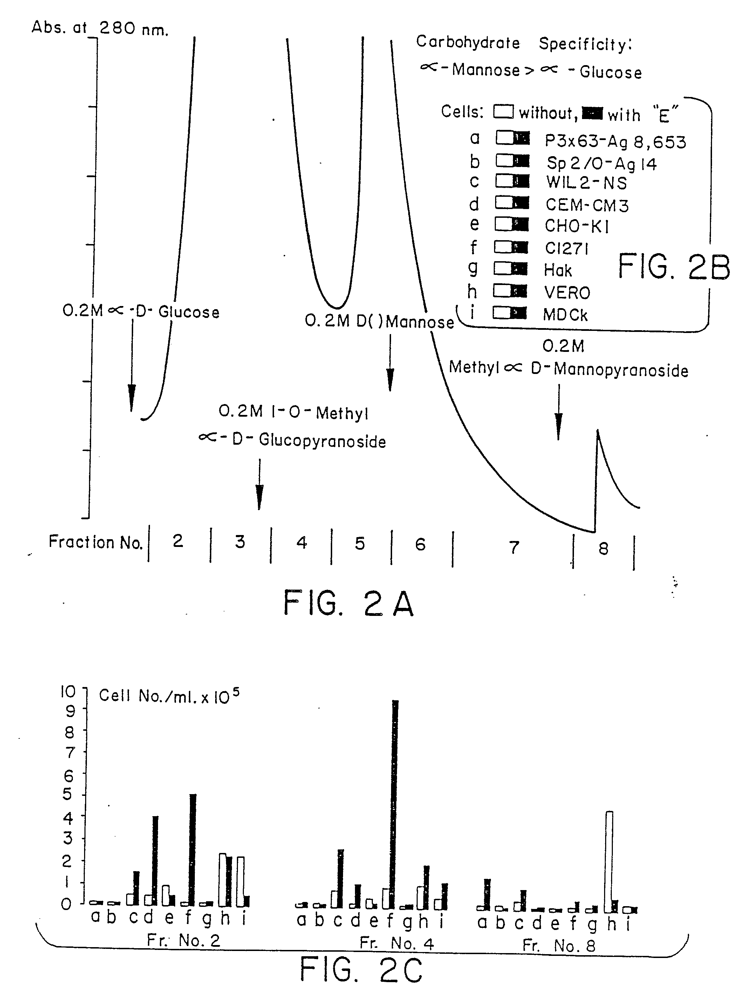

Fig. 2A through Fig. 2C represents the results obtained from the Concanavalin A lectin chromatography. Fig. 2A represents UV light absorption of the chromatogram for a test sample at 280 nm. The graph shows eight fractions, labelled as Fr. No. 1 through Fr. No. 8. These fractions were isolated, collected, dialyzed against physiological saline and .2 micron filtered.

Fig. 2B shows nine different cells lines which were used in the test for growth promotion. The clear portion represents cell growth in media without cell growth enhancer "E", and the darkened field represents cell growth in media with cell growth enhancer "E".

Fig. 2C is a bar graph representing the cell growth of the nine cell lines of Fig. 2B grown with Fr. No. 2, Fr. No. 4, and fr. No. 8 (see Fig. 2A). The cell lines were incubated at 37° C for an appropriate period of time and then the number of cells x$10^5$ per ml. were counted. The cell lines (a through i) in each of Fr. No. 2, Fr. No. 4, and Fr. No. 8 in Fig. 2C from left to right correspond to the cell lines (a through i) in Fig. 2B from top to bottom, respectively. As in Fig. 2B, the light portions of the bar graph in Fig. 2C correspond to cell growth in media without cell growth enhancer "E" and the dark fields correspond to cell growth in media with cell growth enhancer "E".

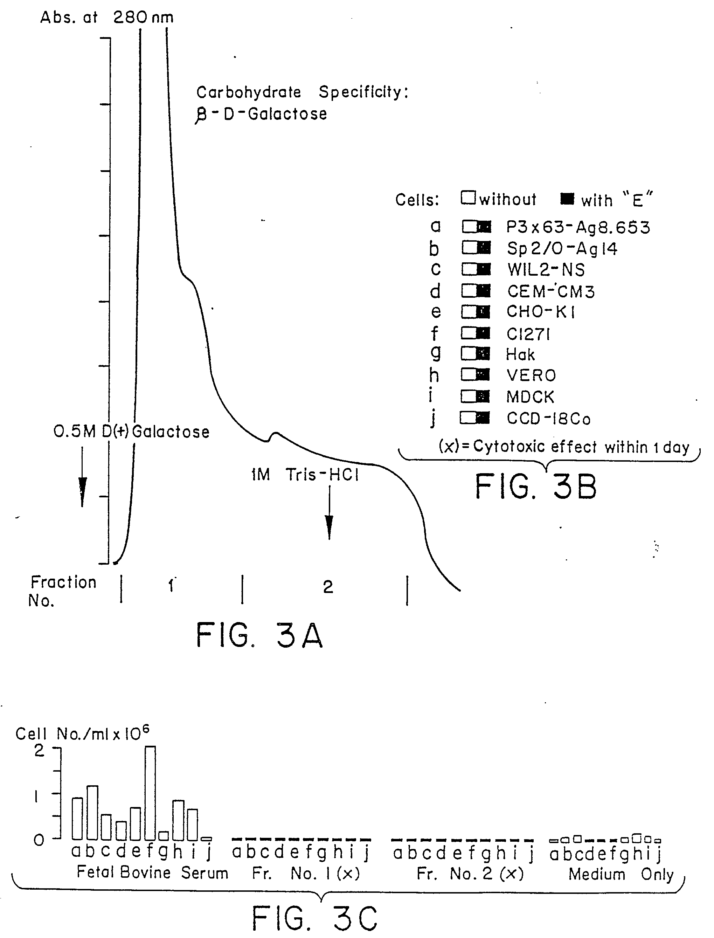

Figure 3A through Figure 3C represent the results obtained from the Castor Bean Lectin chromatography. Figure 3A represents UV light absorption of the chromatogram for a test sample at 280nm. The graph shows two fractions, labelled as Fr. No. 1 and Fr. No. 2. These fractions were isolated, collected, dialyzed against physiological saline and .2 micron filtered.

Figure 3B shows ten different cell lines which were used in the test for growth promotion. The clear portion represents cell grwoth in media without cell enhancer "E", and the darkened filled portion represents cell growth in media with cell growth enhancer "E".

Figure 3C is a bar graph represented the cell growth or lack of growth of the ten cell lines of Figure 3B grown with fetal bovine serum and in the (synthetic) medium only as controls and with Fr. No. 1, and Fro. No. 2 (see Figure 3A). The cell lines were incubated at 37 degrees Centrigrade for an appropriate period of time and then the number of cells x $10^6$ per ml were counted. The cell lines (a through j) of fetal bovine serum, Fr. No. 1, Fr. No. 2 and medium only from left to right correspond to the cell lines (a through j) in Figure 3B from top to bottom, respectively. As in Figure 3B, the light portions of the bar graph in Figure 3C correspond to cell growth in media without cell grwoth enhancer "E" and the dark filled portions correspond to cell growth in media with cell growth enhancer "E".

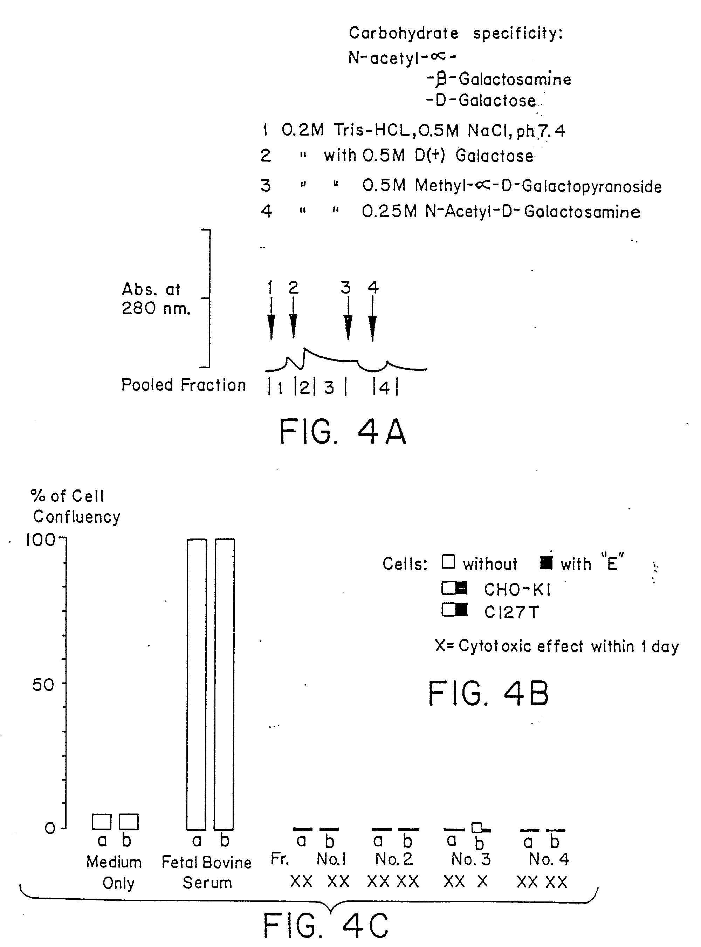

Figure 4A through Figure 4C represent the results obtained from Soybean Lectin chromatography. Figure 4A represents UV light absorption of the chromatogram for a test sample at 280nm. The graph shows four fractions, labelled as Fr. No. 1, Fr. No. 2, Fr. No. 3 and Fr. No. 4. These fractions were isolated, collected, and dialyzed against physiological saline dialyzed and .2 micron filtered.

Figure 4B shows two different cell lines which were used in the test for growth promotion. The clear portion represents cell growth in media without cell growth enhancer "E", and the darkened filled portion represents cell growth in media with cell growth enhancer "E".

Figure 4C is a bar graph representing the cell growth or lack of growth of the two cell lines in Figure 4B grown in (synthetic) medium only and with fetal bovine serum as controls and with Fr. No. 1, Fr. No. 2, Fr. No. 3 and Fr. No. 4 (see Figure 4A). The cell lines were incubated at 37 degrees Centigrade for an appropriate period of time and then the number of cells x $10^6$ per ml were counted. The cell lines (a and b) in each of medium only, fetal bovine serum, Fr. No. 1, Fr. No. 2, Fr. No. 3 and Fr. No. 4 from left to right correspond to the cell lines (a and b) in Figure 4B from top to bottom, respectively. As in Figure 4B, the light portions of the bar graph in Figure 4C correspond to cell growth in media without cell growth enhancer "E" and the dark filled portions correspond to cell growth in media with cell growth enhancer "E".

## Process for obtaining Cell Growth Modulators:

The process utilizes lectins (proteins which specifically and reversibly bind to certain carbohydrate moieties) for affinity binding of the specific growth factors from the biological fluid. Suitable lectins are usually prepared from plant material as, e.g., wheat germ lectin, castor bean lectin, red kidney bean lectin, Concanavaline A, soybean lectin, pea lectin, etc. The lectin can be applied in free or immobilized (for chromatography) form to bind the specific carbohydrate(s). The factor(s) is eluted using lectin specific inhibitory sugar(s) at increasing, or at high concentration, and/or by increasing the ionic strength, varying the pH of the solution, or the addition of denaturants. In some cases borate is used, or the combinations of some of the above variables. The factor or cell growth modulator absorbs light at 280 nm wavelength. The cell growth modulator(s) (activator or inhibitor) can be lyophilized. The cell growth modulator(s) is applied in a physiological solution.

## Process for obtaining Cell Growth Enhancer E:

Erythrocyte (red blood cell) preparation, Enhancer "E", is obtained by disrupting the cells by freezing and thawing, or by suspending (diluting) in distilled water or by using any other conventional or suitable methods. After centrifugation, the extract supernatant in physiological solution is combined with Cell Growth Activators for various applications.

The Enhancer "E" alone has little or no cell growth promoting activity alone but in combination with the Cell Growth Activators obtained with lectins or other methods it usually enhances the growth of certain cells several fold.

Specific lectins specifically bind different kinds of carbohydrates and also those in the oligosaccharide chain which is part of or attached to a glycosylated protein or glycoprotein. This carbohydrate specific binding provides information about the carbohydrate determinants of the oligosaccharides and the structural requirements for the binding, e.g. the configurational specificity of the carbohydrate in the carbohydrate moiety of glycoproteins. The binding by specific lectin and the elution of the Cell Growth Activators or Inhitbitors by lectin binding site specific carbohydrates identifies them as glycoproteins, with the characteristic carbohydrate moiety and its structural organization being essential for the binding. This specificity provides the basis to differentiate the various growth modulators and helps define the ceil type specific biological activities. It is a sensitive method for characterizing the different cell growth activators and

4

inhibitors based on a relationship between their lectin specific carbohydrate determinant(s) and the specificity of their cell growth promoting or inhibitory activity.

The following data relate to specific examples of cell growth promoting and inhibitory materials depicting the methodology and apparatus. This data is set forth for example only, and is not meant to be limiting on the instant invention.


Example I. Wheat Germ Lectin Chromatography

(Carbohydrate Specificity: Tri-N-acetyl-D-Glucosamine, N-acetylneuraminic Acid).

A slurry of commercially available Wheat Germ Agarose was introduced into chromatographic column and was equilibrated with the starting buffer. (In this example 0.02M Tris-HCl buffer with 0.1M NaCl, pH 7.4). The applied biological fluid (in this example, plasma derived serum), dialysed against the starting buffer, was introduced into the column. After application of the material, the column was washed with the starting buffer to remove any non-specifically absorbed material. It was followed with 0.5M NaCl in the same buffer to elute weakly adsorbed material, and when the absorption at 280nm of the eluent became negligible, it was continued with 0.5M N-Acetyl-D-Glucosamine in the same buffer. The graph (Fig. 1A) indicates relatively high absorption at 280nm in the N-Acetyl-D-Glucosamine eluted fraction. The fractions were dialysed against 0.85% NaCl solution and passed through a sterile 0.2 micron membrane filter.

Approximately 9% of the sterile samples were added to synthetic media (used routinely for the specific cell) alone and together with about 1% (v/v) of the Enhancer "E" (see Example V), and tested for cell growth promoting activity with different cell types (Fig. 1B).

The cell growth promoting activity was evaluated after the appropriate incubation period at 37° C., by counting the cells using a hemacytometer.

The graph (Fig. 1C) indicates the marked difference of cell type specific activities in the two fractions. It also indicates the marked effect of Enhancer Preparation "E" from Red Blood Cells on the activity of some cells.


Example II. Concanavalin A Lectin Chromatography

(Carbohydrate specificity: D-Mannose, D-Glucose, and sterically related sugars).

A slurry of commerically available Concanavaline A-Sepharose was introduced into chromatographic column and was equilibrated with starting buffer. (In this Example 0.02M Tris-HCl buffer with 0.5M NaCl, pH 7.4). The applied biological fluid (In this example plasma derived serum), dialysed against the starting buffer was introduced into the column. After application of the material, the column was washed with the starting buffer to remove nonadsorbed material. It was followed stepwise, as the absorption at 280nm decreased, with the specific sugars in increasing affinity to Concanavalin A to elute specifically adsorbed materials. The graph (Fig. 2A) indicates high absorption at 280nm after the application of Glucose, Methyl-Glucopyranoside and much lower after Methyl-Mannopyranoside. The fractions were dialysed against 0.85% NaCl solution and filtered through 0.2 micron sterilized membrane.

The filtered samples were added to synthetic media at about 9% (v/v) concentration alone and together with the addition of about 1% (v/v) of Enhancer Preparation "E" (see Example V), and tested for cell growth promoting activity with different cell types (Fig. 2B).

The cell growth promoting activity was evaluated after the appropriate incubation period at 37° C, by counting cells using a hemacytometer. The graph (Fig. 2C) indicates the marked difference of cell type specific activities in the three fractions tested. It also indicates the marked effect of Enhancer "E" from Red Blood Cells on the activity of some cells.


Example III. Castor Bean Lectin - 120 Chromatography

(Carbohydrate specificity: D-Galactose)

A slurry of commercially available castor bean lectin - 120 agarose was introduced into chromatographic column and was equilibrated with starting buffer. ((n this example 0.02M Tris-HCl buffer with 0.5M NaCl, pH 7.40). The applied biological fluid (in this example plasma derived serum), dialysed against the starting buffer was introduced into the column. After application of the material, the column was washed with the starting buffer to removed non-absorbed material. It was followed, as the absorption at 280nm decreased, with the specific sugar having affinity to Castor Bean - 120 to elute specifically absorbed materials. The graph (Figure 3A) indicates high absorption at 280nm after the application of galactose. The collected fractions were dialysed against 0.85% NaCl solution and filtered through 0.2 micron sterilized membrane.

The filtered samples were added to synthetic media alone at about 9% (v/v) concentration and together with the addition of about 1% (v/v) of Enhancer Preparation "E", and tested for cell growth promoting activity with different cell types (Figure 3B).

The cell growth activity was evaluated after the appropriate incubation period at 37 degrees Centigrade, by counting cells using a hemacytometer.

The graph (Figure 3C) indicates the marked difference of activities. The fetal bovine serum supported very good cell growth with all the cells tested except with CCD-18Co cells which grow very slowly. Even in the synthetic medium only control there were surviving cells in six out of ten cases at the termination of the experiment. Applying the D(+) galactose eluted fractions, all the cells were dead within twenty-four hours.

Example IV. Soybean Lectin Chromatography

(Carbohydrate specificity: N-acetyl-D-Galactosamine and D-Galactose).

A slurry of commercially available soybean lectin agarose was introduced into chromatographic column and was equilibrated with starting buffer. (In this example 0.02M Tris-HCl buffer with 0.5M NaCl, pH7). The applied biological fluid (in this example plasma derived serum), dialysed against the starting buffer was introduced into the column. After application of the material, the column was washed with the starting buffer to remove non-absorbed material. It was followed stepwise, as the absorption at 280nm decreased with the specific sugars in increasing affinity to soybean lectin to elute specifically absorbed materials.

The graph (Figure 4A) indicates increased absorption at 280nm after the application of galatose. After the addition of methyl-galactopyranoside the absorption decreased but it increased again after the application of N-Acetyl-Glucosamine. The fractions were dialysed against 0.85% NaCl solution and filtered through 0.2 micron sterilized membrane.

The filtered samples were added to synthetic media alone at about 9% (v/v) concentration and together with addition of about 1% (v/v) enhancer "E" preparation, and tested for cell growth promoting activity with different cell types.

The cell growth promoting activity was evaluated after the appropriate incubation period at 37 degrees Centigrade, by evaluating the confluency of cell sheet formed after fixation and staining.

The graph (Figure 4C) indicates the marked difference of activities. In the control containing synthetic medium only there were surviving cells at the termination of the experiment. The fetal bovine serum supported very good cell growth with both cells tested. Applying the high (0.5M) sodium chloride, the D(+) galactose (two fractions) and the N-Acetyl-D-Galactosamine eluted fractions, all the cells were dead within twenty-four hours.

Example V

The procedure as outlined in Examples I through IV was repeated using pea lectin. A glycoprotein fraction which promoted cell growth was derived upon elution with fucose.

Example VI. Enhancer "E"

Blood containing anticoagulant (e.g. sodium citrate) was centrifuged. The plasma was carefully removed with the top layer of the sedimented cellular materials and platelets. The red blood cell sediment was carefully resuspended in 0.85% sodium chloride solution and centrifuged again. The supernatant was then removed, the red blood cell sediment was resuspended in 0.85% sodium chloride solution and centrifuged. The supernatant was removed and the red blood cell sediment was resuspended with twice of the packed red blood cell volume of 0.85% sodium chloride solution (one volume of red blood cells plus two volumes of 0.85% sodium chloride solution).

The suspension was checked for the absence of platelets. If the red blood cell suspension contained platelets the washing continued. When the red blood cell suspension was clear of platelets, six volumes of distilled water was added to one volume of the packed red blood cells to disrupt the cells. When all the cells were lysed, concentrated sodium chloride solution was added to bring the sodium chloride concentration of the suspension to 0.85% and the dilution of the packed red blood cells to 1:10 (one volume of red blood cells and nine volumes of 0.85% sodium chloride solution).

Alternative method:

To a volume of the packed red blood cells, nine volumes of 0.85% sodium chloride solution were added and the cells were resuspended. The resuspended red blood cells were frozen and thawed three times.

The lysed red blood cell suspension (obtained by a suitable method) was centrifuged. The supernatant was removed and filtered through a sterile 0.2 micron filter. The obtained enhancer "E" was stored frozen at 1:10 dilution.

The following table depicts the cells used in the above examples:

| Cells Used | | |
|---|---|---|
| ATCC No. | | |
| CRL 1581 | SP 2/0 - Ag 14 | Hybridoma, non-secreting Mouse |
| CRL 1580 | P3X63 - Ag 8.653 | Myeloma, non-secreting Mouse |
| CRL 2155 | WIL2-NS | Lymphoblast, non-secreting variant, Human |
| TIB 195 | CEM-CM3 | Acute Lymphoblastic leukemia, Human |
| CCL 61 | CHO K1 | Ovary, Chinese hamster Epithelial - like |
| CCL 34 | MDCK (NBL-2) | Kidney, Canine Canis familiaris Epithelial - like |
| CCL 15 | Hak | Kidney, Syrian hamster, Epithelial - like |
| CCL 81 | VERO | Kidney, African Green Monkey Fibroblast like |
| CRL 1616 | C1271 | Mammary tumor, Mouse, non-transformed |

As can be seen in the graphs (Figs. 1C, 2C, 3C and 4C), different lectin specific cell growth and/or inhibitory materials substantially enhance and inhibit growth of particular cell types. This demonstrates how lectins can be used to isolate selective cell growth promoting and inhibitory materials.

The above results indicate that growth modulating substances which are isolated from body fluids with lectins which specifically bind with D-Glucose, Tri-N-acetyl-D-Glucosamide, N-acetylneuraminic acid, D-Mannose, Fucose and stereoisomers thereof have been found to have cell growth promotian activities. These results also indicate that growth modulating substances which are isolated from body fluids with lectins which specifically bind with D-Galactose, N-acetyl-D-Galactosamine and stereoisomers thereof have been found to have growth inhibiting properties.

These cell growth activators or inhibitors can be useful in a broad range of applications. As described above, they can be used alone or in combination with enhancer "E" to substitute serum in an in vitro cell culture media. They can therefore be used to replace expensive and often scarce media such as fetal bovine serum.

Specific antibodies can also be raised against the Cell Growth Modulators by conventional means, such as polyclonal and monoclonal type (G. Kohler and C. Milstein, 256 Nature 495 (1975)). These antibodies can be useful for separating and further purifying the specific Cell Growth Modulators by immunoprecipitation or immunoaffinity chromatography.

Also, by labelling these specific antibodies directly or indirectly with radioisotopes, enzymes, fluorescent or luminescent compounds, they can be used for diagnosis, detection, quantitative measurements and histochemistry. For example, some researchers have explored a possible relationship between various growth promoting substances and certain diseases, such as cancer. By using the antibodies to measure for the presence and quantity of cell growth mediators, these conditions can be diagnosed.

These cell growth activators and inhibitors are also useful for manipulating cell growth, cell growth activation alone or in specific combinations, depending on the cell type. Further uses include differentiating cells and for the production of biomolecules, e.g. virus vaccines, TPA (tissue plasminogen activator), urokinases and monoclonal antibodies.

Because they are native (essentially unaltered) biological substances having the same or essentially the same function in vitro as in vivo, these cell growth activators and inhibitors can have clinical applications in the diagnosis and treatment of growth defects or hypoplastic diseases, wound healing and tisse regeneration. They can be useful by finding correlations between their presence or absence as well as their concentrations during development and aging, in healthy persons and also persons afflicted with various tumors. Finally, they can be useful in the area of animal health and husbandry by, e.g., increasing and maintaining rapid growth.

**Claims**

1. A method of producing glycoprotein cell growth modulators which comprises the steps of
   (a) reacting a sample of a body fluid with lectins to enable the lectins to bind reversibly to glycoproteins in the body fluid;
   (b) separating the bound lectins/glycoproteins from the remaining body fluid;
   (c) eluting the glycoproteins from the lectins; and
   (d) collecting the glycoproteins having cell growth modulating activity.

2. A method according to claim 1 wherein the body fluid is selected from serum and plasma.

3. A method according to claim 1 or claim 2, wherein the lectins are selected from wheat germ lectin, Concanavalin A, castor bean lectin, soybean lectin, red kidney bean lectin and pea lectin.

4. A method according to claim 3, wherein the lectins are specific for D-glucose, tri-N-acetyl-D-glucosamine, N-acetyl neuraminic acid, D-mannose, fucose and stereoisomers thereof.

5. A method according to claim 3, wherein the lectins are specific for D-galactose, N-acetyl-D-galactosamine and stereoisomers thereof.

6. A cell growth modulator obtained by a method according any one of claims 1 to 5 and which absorbs light at a wavelength of about 280 nm.

7. A cell growth modulator according to claim 6 which is a cell growth promoter obtained according to the method of claim 4.

8. A cell growth modulator according to claim 6 which is a cell growth inhibitor obtained according to the method of claim 5.

9. A culture medium composition for promoting the growth of cells in vitro, which comprises an effective growth promoting amount of the cell growth promoter according to claim 7 together with a synthetic medium.

10. A culture medium composition for inhibiting the growth of cells in vitro, which comprises an effective growth inhibiting amount of the cell growth inhibitor according to claim 8 together with a synthetic medium.

11. A culture medium according to claim 9 further comprising a growth effective amount of a cell growth enhancer "E" produced by disrupting red blood cells and separating the supernatant from the cellular debris.

12. A cell growth enhancer E produced by the process of:
(a) providing for a sample of red blood cells;
(b) disrupting the red blood cells in said sample; and
(c) removing the disrupted red blood cells from said sample;
wherein said enhancer E alone possesses little or no cell growth promoting activity, but when admixed with cell growth activators will enhance the growth of cells by said cell growth activators.

FIG. IA

FIG. IB

FIG. IC

FIG. 2A

FIG. 2B

FIG. 2C

FIG. 3A

Abs. at 280 nm

Carbohydrate Specificity:
β-D-Galactose

0.5M D(+)Galactose

1M Tris-HCl

Fraction No.   1   2

FIG. 3B

Cells:  □without  ■ with "E"
a  P3x63-Ag8.653
b  Sp2/0-Ag14
c  WIL2-NS
d  CEM-CM3
e  CHO-KI
f  CI27I
g  Hak
h  VERO
i  MDCK
j  CCD-18Co

(x) = Cytotoxic effect within 1 day

FIG. 3C

Cell No./ml x $10^6$

abcdefghij     abcdefghij     abcdefghij     abcdefghij
Fetal Bovine Serum   Fr. No. I (x)   Fr. No. 2 (x)   Medium Only

Carbohydrate specificity:

N-acetyl-$\alpha$-

-$\beta$-Galactosamine

-D-Galactose

1  0.2M Tris-HCL, 0.5M NaCl, ph 7.4

2  "  with 0.5M D(+) Galactose

3  "  "  0.5M Methyl-$\alpha$-D-Galactopyranoside

4  "  "  0.25M N-Acetyl-D-Galactosamine

Abs. at 280 nm.

1  2    3  4

Pooled Fraction  |1| |2| 3 |  |4|

## FIG. 4A

% of Cell Confluency

100

50

0

Cells: □ without  ■ with "E"

□■ CHO-KI

□■ C127T

X = Cytotoxic effect within 1 day

## FIG. 4B

|  | a b | a b | a b | a b | a b | a b |
|--|-----|-----|-----|-----|-----|-----|
| | Medium Only | Fetal Bovine Serum | Fr. No.1 | No. 2 | No. 3 | No. 4 |
| | | | XX XX | XX XX | XX X | XX XX |

## FIG. 4C